# EUROPÄISCHE PATENTSCHRIFT

(11) **EP 3 064 158 B1**
(45) Veröffentlichungstag und Bekanntmachung des Hinweises auf die Patenterteilung: **17.08.2022**
(21) Anmeldenummer: 15157264.1
(22) Anmeldetag: 03.03.2015
(51) Int. Cl.: A61B 17/3201, A61B 18/14, A61B 17/00

(54) **Medizinische Vorrichtung mit Wasserstrahlapplikator und Verfahren zur Aufrüstung einer chirurgischen Schere**
Medical device with water jet applicator and method for upgrading a surgical scissors
Dispositif médical doté d'un applicateur de jeu d'eau et procédé destiné à équiper des ciseaux chirurgicaux

(43) Veröffentlichungstag der Anmeldung: 07.09.2016
(73) Patentinhaber: Erbe Elektromedizin GmbH, 72072 Tübingen (DE)
(72) Erfinder: Fech, Andreas, 72072 Tübingen (DE); Fischer Klaus, 72202 Nagold (DE); Enderle, Markus D., 72070 Tübingen (DE)
(74) Vertreter: Bohnenberger, Johannes

(56) Entgegenhaltungen:
- WO-A1-2005/104965
- DE-A1-102006 027 873
- DE-B3-102007 044 325
- US-A- 5 624 393
- US-A- 6 030 356
- US-A1- 2003 139 743
- US-A1- 2007 255 272
- US-A1- 2013 079 751

## Beschreibung

Die Anmeldung beschreibt einen Wasserstrahlapplikator für den Transport und die Abgabe eines Fluids, eine medizinische Vorrichtung mit einem entsprechenden Wasserstrahlapplikator sowie ein Verfahren zur Aufrüstung einer chirurgischen Schere.

Aus der Laparoskopie sind elektrochirurgische Instrumente zum Schneiden und/oder Koagulieren bekannt. Entsprechende HF-Zangen werden über Endoskope eingebracht.

Aus der DE 10 2006 027 873 A1 ist es bekannt, entsprechende elektrochirurgische Zangen mit einem Wasserstrahlapplikator auszustatten. Dieser kann über einen Führungskanal am distalen Ende des Instruments aus- und wieder eingefahren werden.

Die WO 2005/104965 A1 beschreibt ebenfalls einen Applikator für die Wasserstrahl-Chirurgie. Die US 2007/0255272 A1 betrifft ein Schlauchset zur Befestigung an einem elektrochirurgischen Instrument mit einem oder mehreren Zinken, um das Instrument um eine Spülfunktion zu erweitern. Die US 6 030 356 A offenbart eine verbesserte Spülvorrichtung für motorisierte chirurgische Werkzeuge. Die US 2003/139743 A1 betrifft verbesserte bipolare chirurgische Pinzetten mit spülbaren Spitzen. Die US 5 624 393 A bietet einen nachrüstbaren Aufsatz für eine Knochensäge oder -bohrmaschine zur Kühlung des Knochens während der Schneid- und/oder Bohrphase der Operation. Die US 2013/079751 A1 offenbart ein System zum Befestigen eines Spülsystems an einer Knochensäge, um Spülflüssigkeit u.a. zur Kühlung zu einer zu operierenden Stelle zu leiten.

Die DE 10 2007 044325 B3 betrifft eine chirurgische Schere mit einem proximalen und einem distalen Ende, wobei eine Spülflüssigkeit einfach und sicher zu einem distalen Ende der Schere hingeleitet werden kann, indem auf einer Außenfläche der Schere ein Fluidkanal ausgebildet ist, der sich über einen Teilabschnitt der Schere zwischen dem proximalen und dem distalen Ende erstreckt und der mindestens auf einem Teil seiner Länge seitlich geöffnet ist.

Bei der Applikation des Wasserstrahls mittels eines entsprechenden Wasserstrahlapplikators wird mit einer hinreichend hohen Geschwindigkeit ein Fluid, insbesondere eine Flüssigkeit, in Gewebe eingebracht. Das Gewebe nimmt die Flüssigkeit vorübergehend auf, wobei sich diese in dem extrazellulären Raum einlagert, so dass es in der Nähe der Injektionsstelle zu einer lokalen Volumenzunahme des Gewebes kommt. Wird der Wasserstrahl zwischen zwei eng benachbarten Strukturen, beispielsweise zwischen der Leber und der Gallenblase, abgegeben, so bewirkt die Volumenzunahme, dass das dazwischen liegende Bindegewebe aufquillt und so den Abstand zwischen den Organen vergrößert. Das Fluid-Schutzkissen kann ein Vielfaches des Abstands im Ausgangszustand betragen und bietet so einen erhöhten thermischen und mechanischen Schutz der Organe.

Bei dem Instrument der DE 10 2006 027 873 A1 erweist sich die Aufbereitung als ein Problem. Des Weiteren kann dieses Instrument nur für bestimmte Anwendungen eingesetzt werden.

Ausgehend von der DE 10 2006 027 873 A1 stellt sich die vorliegende Erfindung die Aufgabe, einen verbesserten und/oder anderen Ansatz zur Ausstattung eines Instruments mit einem Wasserstrahlapplikator bereitzustellen. Insbesondere sollen andere Anwendungsgebiete erschlossen werden.

Diese Aufgabe wird durch die medizinische Vorrichtung gemäß Anspruch 1 und das Verfahren gemäß Anspruch 14 gelöst. Die weiteren Ausführungsformen werden in den Nebenansprüchen definiert.

Insbesondere wird die Aufgabe durch eine medizinische Vorrichtung gelöst, die einen Wasserstrahlapplikator für den Transport und die Abgabe eines Fluids und ein Instrument umfasst. Der Wasserstrahlapplikator umfasst:
- eine Applikatorleitung;
- einen Fluidanschluss zur Verbindung der Applikatorleitung mit einer Fluidquelle;
- einen Applikatorauslass zur Abgabe des Fluids und mindestens zwei Befestigungseinrichtungen zur lösbaren Befestigung des Wasserstrahlapplikators an einem Instrument.

Die Applikatorleitung umfasst einen ersten Abschnitt mit einem ersten Elastizitätsmodul und einem ersten Durchmesser und einen zweiten Abschnitt mit einem zweiten Elastizitätsmodul und einem zweiten Durchmesser, wobei sich das erste Elastizitätsmodul vom zweiten Elastizitätsmodul unterscheidet, wobei mindestens eine erste Befestigungsvorrichtung, nämlich eine Medialbefestigungsvorrichtung, eine fluide Verbindung zwischen dem ersten Abschnitt und dem zweiten Abschnitt herstellt. Das Instrument umfasst eine erste Branche und eine zweite Branche, wobei die Branchen über mindestens ein Gelenk miteinander verbunden und mittels eines ersten Handgriffs bzw. eines zweiten Handgriffs betätigbar sind, wobei mindestens eine zweite Befestigungsvorrichtung eine Distalführung an einer der Branchen zur Aufnahme des Applikatorauslasses und eines Abschnitts der Applikatorleitung umfasst.

Dabei wird unter einem Fluid jegliche flüssige oder gasförmige Substanz und deren Mischungen verstanden.

Ein Kern der vorliegenden Erfindung besteht also darin, einen Wasserstrahlapplikator bereitzustellen, der sich auf einfache und effiziente Weise an einem Instrument befestigen lässt. Die Befestigung soll sehr einfach ausgestaltet sein, so dass sich die Verbindung zu dem Instrument ohne die Verwendung von Werkzeugen herstellen lässt. Des Weiteren soll die Befestigung lösbar sein, so dass der Wasserstrahlapplikator nach der Durchführung einer Anwendung von dem Instrument abgenommen werden kann, ohne dass dieses beschädigt wird.

Vorzugsweise umfasst die Befestigungsvorrichtung mindestens ein flexibles Element, so dass eine Art Klippverbindung bzw. Rastverbindung zu dem Instrument hergestellt werden kann. Beispielsweise kann die Befestigungsvorrichtung einen Befestigungsklipp umfassen.

In einer bevorzugten Ausgestaltung handelt es sich bei dem Instrument um eine chirurgische Schere. Die chirurgische Schere kann eine mechanische oder elektrochirurgische Schere (bipolar oder monopolar) sein.

Die Applikatorleitung umfasst einen ersten Abschnitt mit einem ersten Elastizitätsmodul und einen zweiten Abschnitt mit einem zweiten Elastizitätsmodul, wobei sich der erste Elastizitätsmodul von dem zweiten Elastizitätsmodul unterscheidet.

Vorzugsweise weist der erste Abschnitt einen deutlich kleineren Außendurchmesser als der zweite Abschnitt auf, so dass die Geometrie des Instruments am distalen Ende durch den ersten Abschnitt nicht oder nur geringfügig verändert wird, insbesondere nicht vergrößert wird und die mechanischen und elektrischen Eigenschaften der Instrumentenspitze erhalten bleiben. Es ist daher vorteilhaft den ersten Abschnitt der Applikatorleitung aus einem Material mit hohem Elastizitätsmodul und hoher Festigkeit z.B. einer Metalllegierung herzustellen, da hierbei ein Kapillarrohr mit geringer Wanddicke und somit einem geringen Außendurchmesser verwendet werden kann. Zudem wird durch eine hohe Steifigkeit des metallischen Materials erreicht, dass der Applikator bezüglich des Instruments einfach ausgerichtet und positioniert werden kann. Zum Beispiel kann der erste Abschnitt der Applikatorleitung eine vorgegebene Form und Ausrichtung haben, die eine definierte Ausrichtung und Positionierung des Applikatorauslasses am Instrument ermöglicht. Der zweite Abschnitt kann z.B. aus einem Polymer, insbesondere einem Thermosplast, hergestellt werden, da der Außendurmesser und somit die Wandstärke größer gewählt werden können. Weiterhin wird die Flexibilität der Applikatorleitung durch die Verwendung des Polymers deutlich erhöht, so dass er mechanische Bewegungen des Instruments ohne Weiteres mitmacht. Die Ausgestaltung des zweiten Abschnittes ermöglicht es, dass Gelenke des Instruments ohne Weiteres überbrückt werden können. Zusätzlich stellt der zweite Abschnitt der Applikatorzuleitung eine elektrische Isolation dar und verhindert somit ungewünschte elektrische Ströme.

Der Übergang von dem ersten Abschnitt zu dem zweiten Abschnitt kann auf Höhe mindestens einer Befestigungsvorrichtung erfolgen. In einer Ausführungsform wird innerhalb der Befestigungsvorrichtung eine fluide Verbindung zwischen dem ersten und dem zweiten Abschnitt hergestellt. Beispielsweise kann der erste Abschnitt in den zweiten Abschnitt oder der zweite Abschnitt in den ersten Abschnitt hineingesteckt sein, wobei Teile der Befestigungsvorrichtung eine Außenmanschette und eine Führung bilden. Dies ist besonders vorteilhaft, wenn die Befestigungsvorrichtung in einem Spritzgußverfahren hergestellt wird.

Die Befestigungsvorrichtung ist am Instrument ortsfest gehalten. Dadurch ist sichergestellt, dass beim Öffnen und Schließen das distale Ende des ersten Abschnitts keine Relativbewegung zum distalen Ende des Instruments macht. Beispielsweise kann die Befestigungsvorrichtung einen pilzförmigen Fortsatz haben, der sich komprimieren lässt, so dass dieser in eine entsprechend Aussparrung einschnappt. Zur ortsfesten Lagerung kann der Fortsatz der Befestigungsvorrichtung formschlüssig mit einer Branche des Instruments verbunden sein.

In einer Ausführungsform ist zumindest die Befestigungsvorrichtung (z.B. eine Medialbefestigungsvorrichtung) dazu ausgebildet, mit dem Instrument eine ortsfeste Verbindung herzustellen, die auch die fluide Verbindung zwischen dem ersten und dem zweiten Abschnitt herstellt.

In einer Ausführungsform ist der erfindungsgemäße Wasserstrahlapplikator als Einweg-Applikator ausgebildet.

Medizinische Instrumente müssen steril sein. Es ist äußerst problematisch Wasserstrahlapplikatoren herzustellen, die sich nach einer erstmaligen Verwendung einfach sterilisieren lassen. Dies liegt beispielsweise daran, dass verwendete Leitungen und Düsen sehr kleine Durchmesser haben. Erfindungsgemäß ist es vorgesehen, dass das Instrument in einfacher Weise klinisch aufbereitbar ist. Nach einer Anwendung kann also das Instrument sterilisiert und der verwendete Wasserstrahlapplikator entsorgt werden. Dies reduziert das Risiko der Infektionen. Der erfindungsgemäße Wasserstrahlapplikator kann beispielsweise mit einer Düse ausgestattet sein, die einen Durchmesser von weniger als 0,5 mm, insbesondere von weniger als 0,2 mm hat. Beispielsweise kann sich der Düsendurchmesser auf 0,12 mm belaufen. Die Düse kann erfindungsgemäß in das distale Ende der Applikatorleitung integriert, beispielsweise eingeschweißt, sein. Dies ermöglicht eine kosteneffiziente Herstellung des erfindungsgemäßen Wasserstrahlapplikators.

In einer Ausführungsform ist der Wasserstrahlapplikator über die mindestens eine Befestigungsvorrichtung mit dem Instrument verbunden.

Es ergeben sich ähnliche Vorteile, wie diese bereits anhand des Wasserstrahlapplikators erläutert wurden.

In einer Ausführungsform besteht eine lösbare Verbindung zwischen dem Instrument und dem Wasserstrahlapplikator an mindestens zwei Punkten. Gegebenenfalls kann eine Verbindung an drei Punkten bzw. Bereichen hergestellt werden. Die Mehrpunktverbindung stellt sicher, dass der Wasserstrahlapplikator sicher an dem Instrument befestigt ist. Des Weiteren erhöht diese den Handhabungskomfort des Instruments, das mit dem Wasserstrahlapplikator ausgestattet ist.

Das Instrument umfasst eine erste Branche und eine zweite Branche, wobei die Branchen über mindestens ein Gelenk miteinander verbunden und mittels eines ersten bzw. eines zweiten Handgriffs betätigbar sind. Die bereits beschriebene Mehrpunktverbindung hat besondere Vorteile, wenn das Instrument Mittel zum Fassen und/oder Durchtrennen von Gewebe aufweist.

Mindestens eine der erfindungsgemäßen Befestigungsvorrichtungen umfasst eine Führung. Vorzugsweise ist diese Führung an einer der Branchen, beispielsweise der zweiten Branche vorgesehen. Diese Führung kann den Applikatorauslass und/oder einen Abschnitt der Applikatorleitung aufnehmen. Vorzugsweise handelt es sich bei der zweiten Branche um die Branche, die bei einer sachgemäßen Handhabung unten liegt. Eine Führung an bzw. in einer der Branchen hat den Vorteil, dass der Wasserstrahlapplikator, insbesondere dessen Auslass zumindest in lateraler Richtung (quer zur Längsrichtung der jeweiligen Branche) eine feste Position einnimmt. Die Handhabung der erfindungsgemäßen medizinischen Vorrichtung gestaltet sich also äußerst einfach, wobei eine feste Positionierung des Wasserstrahlapplikators, insbesondere des Auslasses, an der Branche gegeben ist.

In einer Ausführungsform sind die Befestigungsvorrichtungen, dazu ausgebildet, den Applikatorauslass in einer definierten Position relativ zur zweiten Branche zu halten. Vorzugsweise folgt also eine axiale sowie auch laterale Fixierung des Wasserstrahlapplikators relativ zur zweiten Branche. Die Fixierung kann durch die Führung und/oder durch das Zusammenwirken mehrerer Befestigungsvorrichtungen bewerkstelligt werden.

Für die Sterilisation des Instruments kann es vorteilhaft sein, wenn die Führung Seitenöffnungen aufweist. Die Führung kann beispielsweise einen Kanal umfassen, der lediglich teilweise geschlossen ist. Der Kanal kann durch das Einsetzen einer entsprechenden Hülse oder durch das Vornehmen einer Bohrung erstellt werden.

In einer Ausführungsform ist die Länge des Kanals deutlich kürzer als eine Branchenlänge der zweiten Branche. Die Branchenlänge kann als Distanz zwischen Spitze der Branche und Rotationsachse des Gelenks mittels derer die Branchen verbunden sind definiert werden. Die Länge des Kanals kann maximal 80 % der Branchenlänge oder maximal 50 % der Branchenlänge betragen. In einem Ausführungsbeispiel ist die Länge des Kanals kleiner als 4 cm, insbesondere kleiner als 3 cm, insbesondere kleiner als 2 cm, insbesondere kleiner als 1 cm.

Das Instrument kann als Befestigungsvorrichtung eine Führungshülse an einem der Handgriffe umfassen. Die Führungshülse kann aus Kunststoff hergestellt sein. Die Führungshülse dient zur Befestigung des Wasserstrahlapplikators am Instrument und ist vorzugsweise derart ausgebildet, dass der Wasserstrahlapplikator in die Führungshülse eingeführt werden kann. Alternativ oder zusätzlich kann die Führungshülse derart ausgebildet sein, dass sie sich auf den Handgriff aufklippen oder diesen umschließend anklippen lässt. Die Führungshülse kann mindestens einen Abschnitt der Applikatorleitung aufnehmen oder fest mit dieser verbunden sein. Die Führungshülse ermöglicht ein schnelles und einfaches Befestigen des Wasserstrahlapplikators an dem Instrument. Die Führungshülse kann so dimensioniert sein, dass sie sich einfach sterilisieren lässt. In einer Ausführungsform ist die Führungshülse Bestandteil eines Einweg- Wasserstrahlapplikators.

Die mindestens eine Befestigungsvorrichtung kann einen ersten und einen zweiten Anschlag umfassen, um die Applikatorleitung vorgespannt am Instrument zu halten. Insbesondere in Kombination mit der bereits beschriebenen Führungshülse lässt sich eine effektive Halterung des Wasserstrahlapplikators dadurch erzielen, dass zumindest ein Teil der Applikatorleitung zwischen zwei Anschlägen vorgespannt ist. Vorzugsweise ist die Applikatorleitung hierfür mit korrespondierenden Anschlägen ausgestattet. Die Anschläge an dem Instrument können sich im proximalen und im distalen Bereich befinden, wobei die Führungshülse vorzugsweise zwischen dem distalen Bereich und dem proximalen Bereich angeordnet ist.

Das Gelenk des Instruments kann ein Drehgelenk mit einer Rotationsachse sein, wobei eine der Befestigungsvorrichtungen, vorzugsweise die Medialbefestigungsvorrichtung nahe bei oder auf der Rotationsachse des Drehgelenks angeordnet ist. Anmeldungsgemäß kann nahe bei so verstanden werden, dass die Befestigungsvorrichtung maximal 4 cm, insbesondere maximal 3 cm, insbesondere maximal 1 cm von der Rotationsachse entfernt angeordnet ist. Eine Anordnung mindestens einer Befestigungsvorrichtung nahe bei oder auf der Rotationsachse hat den Vorteil, dass der Wasserstrahlapplikator, insbesondere die Applikatorleitung, vorteilhaft entlang des Instruments geführt werden kann, wobei auch ein Öffnen oder Schließen des Instruments möglich ist, ohne dass die Applikatorleitung absteht.

Das Instrument kann eine Schere sein, wobei die erste Branche ein erstes Scherenblatt und die zweite Branche ein zweites Scherenblatt umfasst. In einer Ausführungsform handelt es sich bei der Schere um eine gekrümmte Schere, die ein gekrümmtes erstes Scherenblatt hat, wobei das zweite Scherenblatt korrespondierend zu dem ersten Scherenblatt ebenfalls gekrümmt ausgebildet ist. Das Instrument kann eine elektrochirurgische Schere mit mindestens einem HF-Anschluss sein. In einer Ausführungsform befindet sich eine Befestigungsvorrichtung nahe bei dem HF-Anschluss, zumindest auf dem Handgriff an dem auch der HF-Anschluss vorgesehen ist.

In einer Ausführungsform umfasst die elektrochirurgische Schere eine Isolierung, die zumindest eines der Scherenblätter teilweise isoliert. Die Applikatorleitung kann mit einer entsprechenden Isolierung ausgestattet sein, so dass ungewünschte Stromflüsse vermieden werden.

Die Befestigungsvorrichtung kann eine Proximalbefestigungsvorrichtung zur Herstellung einer Klippverbindung zu einem der Handgriffe umfassen. Soweit es sich um ein elektrochirurgisches Instrument handelt, ist die Proximalbefestigungsvorrichtung vorzugsweise nahe eines entsprechenden HF-Anschlusses angeordnet. Beispielsweise kann die Befestigungsvorrichtung auf der Höhe eines Scherenauges oder nahe einem Scherenauge angeordnet sein.

Die eingangs genannte Aufgabe wird des Weiteren durch ein Verfahren zur Aufrüstung einer chirurgischen Schere zur Herstellung einer erfindungsgemäßen medizinischen Vorrichtung gelöst, wobei das Verfahren die folgenden Schritte umfasst:
- Verbinden eines Applikatorauslasses eines Wasserstrahlapplikators mit einer Befestigungseeinrichtung der Schere, wobei die Befestigungseeinrichtung eine distale Öffnung umfasst;
- Anklippen einer Applikatorleitung, vorzugsweise nahe einem Drehgelenk der Schere und/oder auf Höhe eines Scherenauges an die Schere,
   wobei die Applikatorleitung umfasst:
      - einen ersten Abschnitt mit einem ersten Elastizitätsmodul und einem ersten Durchmesser;
      - einen zweiten Abschnitt mit einem zweiten Elastizitätsmodul und einem zweiten Durchmesser,
   wobei sich der erste Elastizitätsmodul vom zweiten Elastizitätsmodul unterscheidet, wobei mindestens eine der Befestigungsvorrichtungen, nämlich eine Medialbefestigungsvorrichtung, eine fluide Verbindung zwischen dem ersten Abschnitt und dem zweiten Abschnitt herstellt.

Es ergeben sich ähnliche Vorteile, wie sie bereits in Verbindung mit dem Wasserstrahlapplikator erläutert wurden. Durch das Durchführen des Verfahrens wird eine medizinische Vorrichtung mit den Merkmalen entstehen, wie dies vorab beschrieben wurde.

In einer Ausführungsform umfasst das Verfahren den Schritt des Einführens des Applikatorauslasses in einen Kanal der Schere derart, dass über die distale Öffnung Fluid abgegeben werden kann.

Nachfolgend wird die Erfindung anhand von mehreren Ausführungsbeispielen beschrieben. Hierbei zeigen:
- Fig. 1:: ein erstes Ausführungsbeispiel einer elektrochirurgischen Schere, die dazu ausgebildet ist mit einem Wasserstrahlapplikator verbunden zu werden;
- Fig. 2:: einen Wasserstrahlapplikator zur Verbindung mit der Schere aus Fig. 1 (schematisch dargestellt);
- Fig. 3:: einen Teilschnitt durch die elektrochirurgische Schere gemäß Fig. 1 zusammen mit einem Teilabschnitt des Wasserstrahlapplikators aus Fig. 2;
- Fig. 4:: einen Querschnitt durch den Wasserstrahlapplikator und das Instrument gemäß den Figuren 1 und 2;
- Fig. 5:: ein zweites Ausführungsbeispiel einer elektrochirurgischen Schere;
- Fig. 6:: ein drittes Ausführungsbeispiel einer elektrochirurgischen Schere;
- Fig. 7:: einen Wasserstrahlapplikator zur Anordnung an der elektrochirurgischen Schere gemäß Fig. 6 (schematisch dargestellt);
- Fig. 8:: ein viertes Ausführungsbeispiel einer elektrochirurgischen Schere;
- Fig. 9:: einen Längsschnitt durch den Wasserstrahlapplikator gemäß Fig. 2;
- Fig. 10:: ein fünftes Ausführungsbeispiel einer elektrochirurgischen Schere; und
- Fig. 11:: einen Schnitt durch eine der Branchen der elektrochirurgischen Schere gemäß Fig. 10.

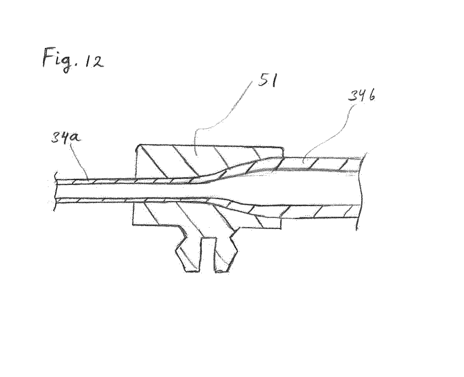

- Fig. 12:: eine Längsschnitt durch ein weiteres Ausführungsbeispiel des Wasserstrahlapplikators.

In der nachfolgenden Beschreibung werden für gleiche und gleichwirkende Teile dieselben Bezugsziffern verwendet.

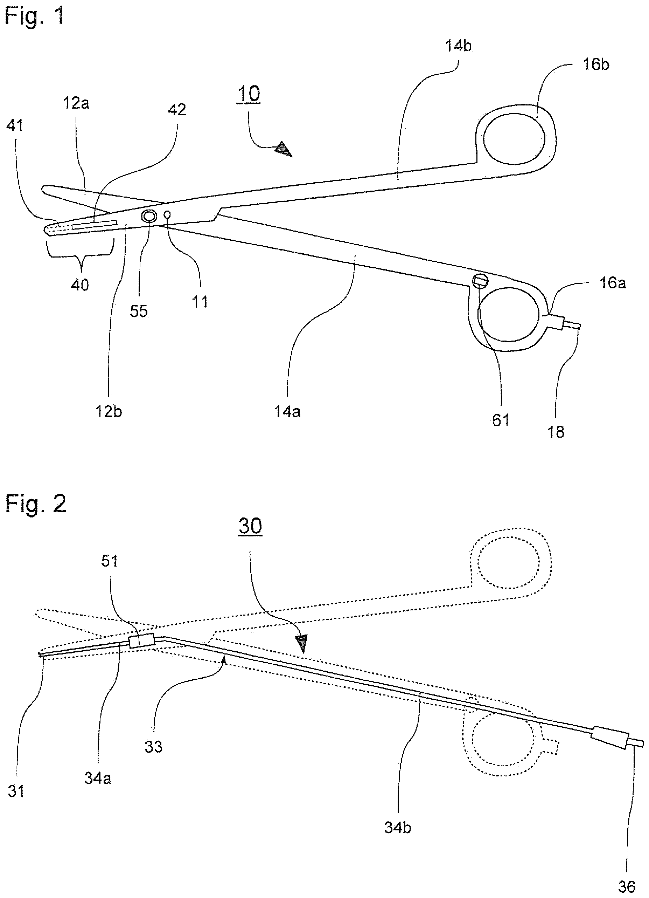

Die Fig. 1 zeigt eine elektrochirurgische Schere 10. Die elektrochirurgische Schere 10 besteht aus einem ersten Teil bzw. ersten Scherenteil, das eine erste Branche 12a und einen ersten Handgriff 14a umfasst, sowie aus einem zweiten Teil bzw. zweiten Scherenteil, das eine zweite, untere Branche 12b und einen zweiten Handgriff 14b umfasst. Am proximalen Ende der Handgriffe 14a, 14b sind jeweils Scherenaugen 16a bzw. 16b zur besseren Aufnahme der Handgriffe 14a, 14b vorgesehen. Bei der elektrochirurgischen Schere 10 gemäß Fig. 1 handelt es sich um ein monopolares Instrument, das an dem ersten Handgriff 14a (am proximalen Ende) einen HF-Anschluss 18 aufweist. Die beiden Teile der elektrochirurgischen Schere 10 sind über ein Scherengelenk 11 miteinander verbunden.

Die elektrochirurgische Schere 10 kann erfindungsgemäß an drei Punkten mit einem Wasserstrahlapplikator 30 (vergleiche Fig. 2) verbunden werden. Hierzu dienen insgesamt drei Befestigungsvorrichtungen. Eine erste Befestigungsvorrichtung wird unter Verwendung einer Distalführung 40, eine zweite unter Verwendung einer Medialbefestigungsvorrichtung 50 und eine dritte unter Verwendung einer Proximalbefestigungsvorrichtung 60 realisiert. Während die erste Befestigungsvorrichtung eine Steckverbindung ist, sind die zweite und dritte Befestigungsvorrichtung Klick- bzw. Klippverbindungen.

Die Distalführung 40 umfasst eine Öffnung an der distalen Spitze der zweiten Branche 12b an die sich ein Führungskanal 41 anschließt, der wiederum in eine Führungsnut 42 mündet. Führungskanal 41 und Führungsnut 42 können in einer Ebene angeordnet sein, die identisch ist mit der Ebene, in der sich die Längsrichtung der zweiten Branche 12b erstreckt. In einer bevorzugten Ausführungsform liegt auch die Rotationsachse des Scherengelenks 11 in dieser Ebene.

Im angeordneten Zustand ist ein Teil des Wasserstrahlapplikators 30 in den Führungskanal 41 eingeführt.

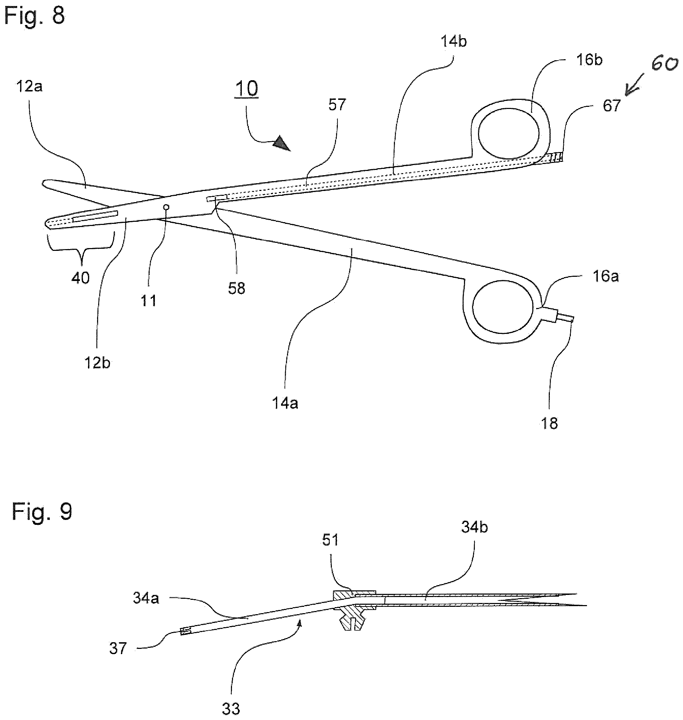

Der Wasserstrahlapplikator 30 (Fig. 2) umfasst an dessen distalen Ende einen Applikatorauslass 31 und an dessen proximalen Ende einen Fluidanschluss 36, der sich mit einer Fluidquelle verbinden lässt. Fluidanschluss 36 und Applikatorauslass 31 stehen über eine Applikatorleitung 33 in Verbindung. In dem Ausführungsbeispiel gemäß Fig. 2 hat die Applikatorleitung 33 zwei Abschnitte. Im distalen Bereich gibt es ein Rohrelement 34a aus Metall, das in eine vorgegebene Form gebogen ist (vgl. z.B. Fig. 9). Der distale Bereich der Applikatorleitung 33 wird durch einen Schlauch 34b gebildet. Das Rohrelement 34a ist in den Schlauch 34b zur Herstellung einer fluiden Verbindung eingesteckt. Die fluide Verbindung wird auf Höhe eines Adapters 51 hergestellt.

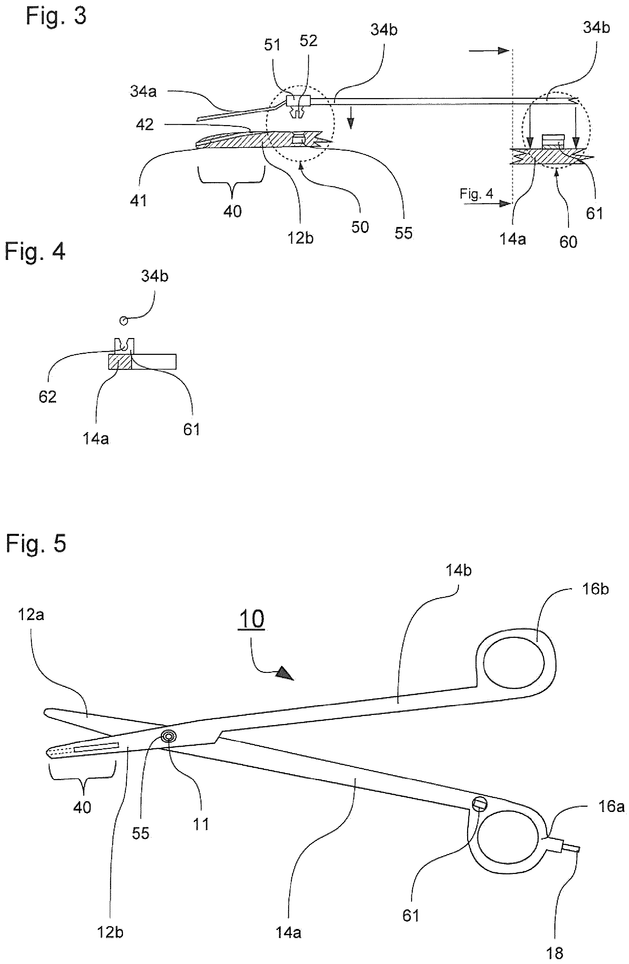

Wie in der Fig. 3 gezeigt umfasst die zweite Befestigungsvorrichtung, nämlich die Medialbefestigungsvorrichtung 50 den Adapter 51 sowie eine an der zweiten Branche 12b nahe dem Scherengelenk 11 angeordnete Aussparung 55. Zur Herstellung der bereits genannten Klickverbindung weist der Adapter 51 auf der der chirurgischen Schere 10 zugewandten Seite einen pilzförmigen Fortsatz 52 auf, der in die korrespondierend ausgebildete Aussparung 55 eingebracht werden kann. Der Fortsatz 52 ist aus einem flexiblen Material hergestellt und derart ausgebildet, dass er sich in die Aussparung 55 einbringen lässt.

In einer anderen Ausführungsform kann die Medialbefestigungsvorrichtung 50 derart ausgebildet sein, dass eine einfache Steckverbindung zwischen dem Adapter 51 und der elektrochirurgischen Schere 10, insbesondere der zweiten Branche 12b hergestellt wird. Aufgrund der Ausgestaltung des Rohrelements 34a unter Verwendung eines federelastischen Materials und der Anordnung der Distalführung 40 und der Medialbefestigungsvorrichtung 50 ist der Applikatorauslass 31 im angeordneten Zustand (vergleiche Fig. 2) sowohl axial als auch lateral fixiert. In der dritten Befestigungsvorrichtung, nämlich der Proximalbefestigungsvorrichtung 60 wird der Schlauch 34b im proximalen Bereich des ersten Handgriffs 14a nahe dem zweiten Scherenauge 16a festgeklippt. Hierfür weist wie anhand der Fig. 3 ersichtlich der erste Handgriff 14a einen vorzugsweise fest integrierten Klipp 61 auf, der die Aufnahme und Führung des Schlauchs 34b ermöglicht. Wie anhand der Figuren 3 und 4 ersichtlich handelt es sich bei dem Klipp 61 um einen Zylinder mit einer nach außen hin offenen Bohrung 62. Fortsätze an den Seitenwänden der seitlich offenen Bohrung 62 halten den Schlauch 34b im eingeklippten Zustand fest.

Der Klipp 61 kann sowohl am ersten als auch am zweiten Handgriff 14a, 14b angeordnet sein. Vorteilhaft ist die Anordnung an dem Handgriff 14a, 14b an dem auch elektrische Anschlüsse, beispielsweise der HF-Anschluss 18, vorgesehen sind. Dies erleichtert die Handhabung der elektrochirurgischen Schere 10. In dem beschriebenen Ausführungsbeispiel ist der Wasserstrahlapplikator 30 abschnittsweise an der zweiten Branche 12b und dem ersten Handgriff 14a angeordnet. Die Applikatorleitung 33 folgt also abschnittsweise der zweiten Branche 12b des zweiten Scherenteils und wechselt dann über zum ersten Handgriff 14a des ersten Scherenteils.

Die Relativbewegung, die beim Öffnen und Schließen der chirurgischen Schere 10 auftritt, kann durch eine axiale Verschiebung des Schlauchs 34b in der Proximalbefestigungsvorrichtung 60 ausgeglichen werden. Vorzugsweise ist daher der Klipp 61 derart ausgestaltet, dass sich der Schlauch 34b innerhalb des Klipps 61 axial leicht bewegen kann.

Alternativ kann der Schlauch 34b mittels der Befestigungsvorrichtungen so geführt sein, dass das überschüssige Schlauchmaterial beim Öffnen der chirurgischen Schere 10 leicht absteht. Durch ein geschicktes Anordnen der Befestigungsvorrichtungen ist es möglich den Anwender hierdurch bei seiner Arbeit, insbesondere bei der Sicht, nicht zu behindern.

Im Unterschied zum ersten Ausführungsbeispiel gemäß den Figuren 1 bis 4 ist die Aussparung 55 für den Adapter 51 der chirurgischen Schere 10 des zweiten Ausführungsbeispiels gemäß Fig. 5 auf der Rotationsachse des Scherengelenks 11 angeordnet. Beispielsweise kann die Aussparung 55 in eine Gelenkschraube, die Teil des Scherengelenks 11 ist, integriert sein. Dies hat zum einen den Vorteil, dass bei der Herstellung der erfindungsgemäßen chirurgischen Schere 10 ein Bearbeitungsschritt an den Scherenteilen wegfällt. Das Vorsehen der Aussparung 55 an dem wesentlich einfacheren Bauteil der Gelenkschraube hat Vorteile. Zum anderen führt das Anordnen des zweiten Befestigungspunkts auf der Rotationsachse des Scherengelenks 11 dazu, dass es beim Öffnen und Schließen der chirurgischen Schere zu einer geringeren Auslenkung des Wasserstrahlapplikators 30, insbesondere des Schlauchs 34b kommt.

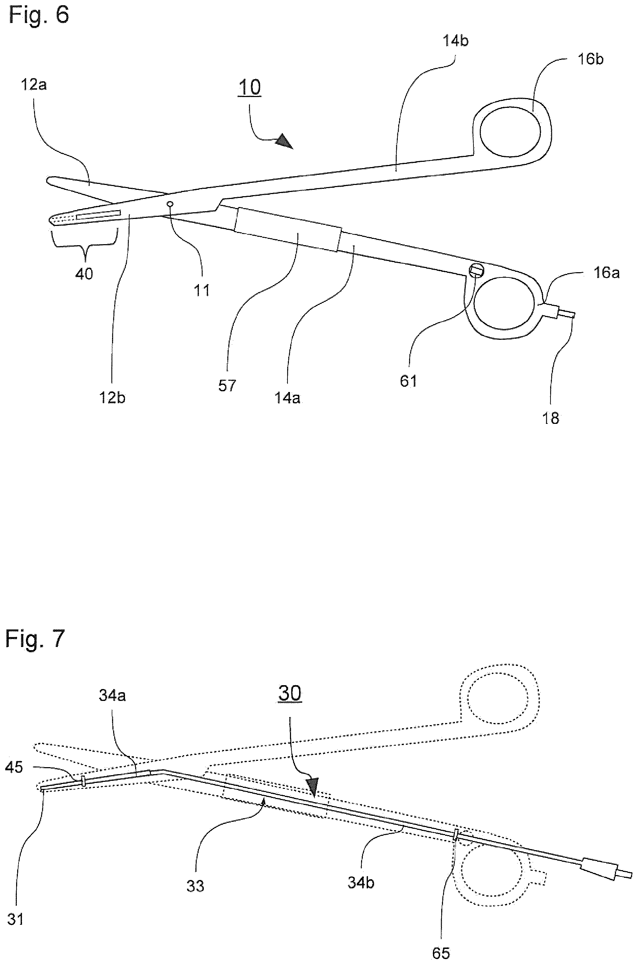

Fig. 6 und 7 zeigt ein drittes Ausführungsbeispiel der erfindungsgemäßen chirurgischen Scheren 10. Anstelle der zweifachen Klippverbindung und der einfachen Steckverbindung der bisher erläuterten Ausführungsbeispiele werden beim dritten Ausführungsbeispiel an zwei Punkten eine Art Steckverbindung und an einem Punkt eine Klippverbindung hergestellt. Gegenüber dem ersten Ausführungsbeispiel unterscheidet sich das dritte Ausführungsbeispiel darin, dass die Medialbefestigungsvorrichtung 50 als eine Führungshülse 57 ausgeführt ist. Diese Führungshülse 57 ist an dem ersten Handgriff 14a angeordnet. Vorzugsweise erfolgt eine Anordnung derart, dass das distale Ende der Führungshülse 57 nahe dem Scherengelenk 11 endet.

In einer bevorzugten Ausführungsform wie diese in dem dritten Ausführungsbeispiel gemäß den Figuren 6 und 7 umgesetzt ist, sind für die axiale Fixierung des Wasserstrahlapplikators 30 zwei Anschlagelemente 45, 65 der Applikatorleitung 33 vorgesehen. Das distale Anschlagelement 45 verhindert eine distale Bewegung des Wasserstrahlapplikators 30 relativ zu der chirurgischen Schere 10, indem das Anschlagelement 45 an der proximalen Öffnung des Führungskanals 41 anliegt. In einer anderen Ausführungsform kann hierfür ein korrespondierendes Anschlagelement an der zweiten Branche 12b vorgesehen werden.

Das proximale Anschlagelement 65 verhindert eine Relativbewegung in proximale Richtung, in dem das proximale Anschlagelement 65 distal des Klipps 61 an diesem anliegt.

Eine bevorzugte Montage des Wasserstrahlapplikators 30 an der chirurgischen Schere 10 kann dadurch erfolgen, dass der Applikatorauslass 31 durch die Führungshülse 57 geschoben und anschließend in die Führungsnut 42 und den Führungskanal 41 eingelegt wird. Hiernach kann der Schlauch 34b in den Klipp 61 eingeklippt werden (vergleiche schematische Darstellung gemäß Fig. 7).

Fig. 8 verdeutlicht eine vierte Ausführungsform der erfindungsgemäßen chirurgischen Schere 10.

Wie auch in der dritten Ausführungsform wird in der vierten Ausführungsform der Wasserstrahlapplikator an zwei Stellen geführt. Eine dritte Befestigung erfolgt anhand einer Schraubverbindung. Des Weiteren ist bei dem vierten Ausführungsbeispiel der Wasserstrahlapplikator 30 ausschließlich entlang des zweiten Scherenteils geführt. Auch die chirurgische Schere 10 der Fig. 8 hat die bereits erläuterte Distalführung 40 an der zweiten Branche 12b. Des Weiteren ist der zweite Handgriff 14b abschnittsweise hohl ausgeführt und dient als Führungshülse 57. Die Führungshülse 57 mündet distal in eine Führungsnut 58 und proximal in einen Fortsatz mit einem Gewinde 67.

Bei der vierten Ausführungsform wird die Proximalbefestigungsvorrichtung 60 im Endeffekt durch das Gewinde 67 an dem proximalen Fortsatz gebildet. Der Wasserstrahlapplikator 30 kann gegen axiale Bewegungen mittels einer Überwurfmutter abgesichert werden, die in das Gewinde 67 eingreift.

Alternativ kann eine Rastverbindung vorgesehen sein. Zusätzlich kann ein Wasserstrahlapplikator 30 für die chirurgische Schere 10 gemäß Fig. 8 ein weiteres Anschlagelement im distalen Bereich auf Höhe der zweiten Branche haben. Dieser Anschlag kann durch den Übergang von dem Schlauch 34b zum Rohrelement 34a realisiert werden. In dieser Ausführungsform hat der Schlauch 34b einen größeren Durchmesser als das Rohrelement 34a. Der Übergang von dem Schlauch 34b zum Rohrelement 34a ist derart gestaltet, dass im angeordneten Zustand das distale Ende des Schlauchs 34b bis an das proximale Ende bzw. die proximale Öffnung des Führungskanals 41 reicht. In dieser Ausführungsform ist der Führungskanal 41 derart dimensioniert, dass dessen Innendurchmesser kleiner ist als der Schlauchdurchmesser. Um das Rohrelement 34a einführen zu können, muss der Durchmesser des Führungskanals 41 jedoch größer als der Durchmesser des Rohrelement 34a sein. Die Verbindungen innerhalb des vorbeschriebenen Anschlagelement müssen fluiddicht und nicht lösbar ausgebildet sein, sodass sie unterschiedlichen Belastungen standhalten.

In einigen Ausführungsformen gibt das Rohrelement 34a des Wasserstrahlapplikators 30 einen Leitungsverlauf vor, so dass das Rohrelement 34a mit einem nur sehr geringen Überstand bis zum Führungskanal 41 und der Führungsnut 42 geführt ist. Im Bereich des Führungskanals 41 insbesondere an dessen distalem Ende ist das Rohrelement 34a komplett in diesen Führungskanal integriert. Fig. 9 zeigt exemplarisch, wie eine entsprechende Verlaufsvorgabe erfolgen kann. Das Rohrelement 34a ist nicht lösbar mit dem Adapter 51 verbunden, wobei beim Austritt des Rohrelements 34a aus dem Adapter 51 eine Ausrichtung vorgegeben ist, die das Rohrelement 34a im angeordneten Zustand hin zur zweiten Branche 12b führt. Dies erleichtert das Einführen des Rohrelements 34a in den Führungskanal 41 und die Führungsnut 42. Es ist auch möglich, dass der Verlauf des ersten Abschnitts des Rohrelements 34a vom Verlauf des Führungskanals 41 geringfügig abweicht. Durch die federelastische Ausbildung des ersten Rohrelements 34a ist dieses gegenüber der zweiten Branche vorgespannt, so dass das Rohrelement 34a nicht aus der Führungsnut 42 herausragt.

Die Applikatorleitung ist immer an der Außenseite des Instruments angeordnet. Mit der Außenseite ist die Branchenseite gemeint, die die Schneidefläche nicht umfasst, bzw. die der Schneidefläche abgewandt ist. Die Außenseite der Branche an der die Applikatorleitung angeordnet ist kann gerade, konvex oder konkav ausgebildet sein. Die Verlaufsform der Applikatorleitung in Verbindung mit dem federelastischen Material und der Verlaufsform der Branche ermöglicht eine vorgespannte Anordnung des ersten Abschnitts der Applikatorleitung im Führungskanal 41.

Der Wasserstrahlapplikator 30 kann an dessen distalen Ende mit einer Düse 37 (vergleiche Fig. 9) ausgestattet sein. Dies trifft nicht nur für den Wasserstrahlapplikator 30 zu, wie dieser in der Fig. 9 gezeigt ist. Auch der Wasserstrahlapplikator gemäß Fig. 2 und Fig. 7 kann in entsprechender Weise ausgebildet sein.

In einer weiteren Ausführungsform kann die Applikatorleitung 33 einstückig, nahtlos aus einem Teil hergestellt werden. Der erste Abschnitt, das Rohrelement 34 a und der zweite Abschnitt, der Schlauch 34 b des Wasserstrahlapplikators 30, wird dann aus einem Material gebildet und weist keine Verbindungsstelle auf. Der Schlauch 34b dieser Applikatorleitung 33 weist an seinem distalen Ende, im Bereich des Adapters 51 eine Verjüngung auf. Die einstückige Herstellung der Applikatorleitung bestehend aus den Abschnitten 34a und 35b gewährleistet eine fluiddichte Verbindung. Die Verjüngung, die Reduzierung des Durchmessers des Abschnitte 34a kann durch Materialumformung bzw. prägen oder ziehen hergestellt werden

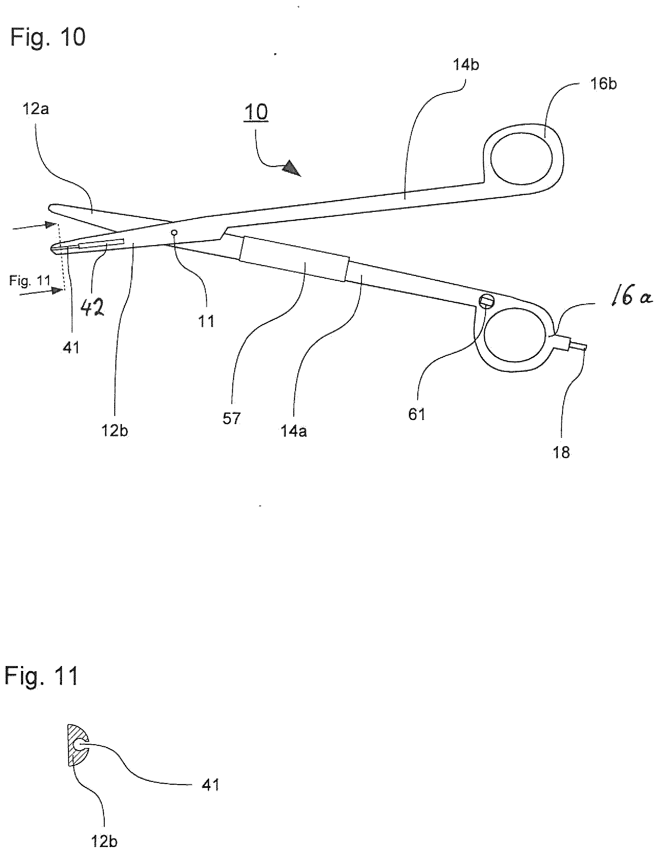

Um die Sterilisation der chirurgischen Scheren 10 zu erleichtern, können alle oder einige der erläuterten Kanäle, beispielsweise der Führungskanal 41 lateral teilweise geöffnet sein. Die Figuren 10 und 11 verdeutlichen eine entsprechende Ausgestaltung des Führungskanals 41. Wie anhand der Fig. 10 ersichtlich, ergibt sich entlang des Verlaufs des Führungskanals 41 eine Nut, die wie in Fig. 11 gezeigt, in die runde Bohrung des Führungskanals 41 mündet. Um einen sicheren Halt des Rohrelements 34a in dem Führungskanal 41 zu gewährleisten, ist der Durchmesser des Führungskanals 41 größer als die lichte Weite der Nut. Mit anderen Worten ist der Durchmesser des Rohrelements 34a größer als die lichte Weite der Nut.

### Bezugszeichenliste

- 10: chirurgische Schere
- 11: Scherengelenk
- 12a, 12b: erste, zweite Branche
- 14a, 14b: Handgriff
- 16a, 16b: Scherenauge
- 18: HF-Anschluss
- 30: Wasserstrahlapplikator
- 31: Applikatorauslass
- 33: Applikatorleitung
- 34a: Rohrelement
- 34b: Schlauch
- 36: Fluidanschluss
- 37: Düse
- 40: Distalführung
- 41: Führungskanal
- 42: Führungsnut
- 45: distales Anschlagelement
- 50: Medialbefestigungsvorrichtung
- 51: Adapter
- 52: Fortsatz
- 55: Aussparung
- 57: Führungshülse
- 58: Führungsnut
- 60: Proximalbefestigungsvorrichtung
- 61: Klipp
- 62: Bohrung
- 65: proximales Anschlagelement
- 67: Gewinde

## Patentansprüche

1. Medizinische Vorrichtung, umfassend:
- ein Instrument (10);
- einen Wasserstrahlapplikator (30) für den Transport und die Abgabe eines Fluids, umfassend:
- eine Applikatorleitung (33);
- einen Fluidanschluss (36) zur Verbindung der Applikatorleitung (33) mit einer Fluidquelle;
- einen Applikatorauslass (31) zur Abgabe des Fluids;
- mindestens zwei Befestigungsvorrichtungen zur lösbaren Befestigung des Wasserstrahlapplikators (30) an einem Instrument (10),
wobei die Applikatorleitung (33) umfasst:
- einen ersten Abschnitt (34a) mit einem ersten Elastizitätsmodul und einem ersten Durchmesser;
- einen zweiten Abschnitt (34b) mit einem zweiten Elastizitätsmodul und einem zweiten Durchmesser,
wobei sich das erste Elastizitätsmodul vom zweiten Elastizitätsmodul unterscheidet,
wobei mindestens eine erste Befestigungsvorrichtung, nämlich eine Medialbefestigungsvorrichtung (50), eine fluide Verbindung zwischen dem ersten Abschnitt (34a) und dem zweiten Abschnitt (34b) herstellt,
wobei das Instrument (10) eine erste Branche (12a) und eine zweite Branche (12b) umfasst, wobei die Branchen (12a, 12b) über mindestens ein Gelenk (11) miteinander verbunden und mittels eines ersten Handgriffs (14a) bzw. eines zweiten Handgriffs (14b) betätigbar sind,
wobei mindestens eine zweite Befestigungsvorrichtung eine Distalführung (40) an einer der Branchen (12a, 12b) zur Aufnahme des Applikatorauslasses (31) und eines Abschnitts der Applikatorleitung (33) umfasst.

2. Medizinische Vorrichtung nach Anspruch 1,
**dadurch gekennzeichnet,**
**dass** der erste Abschnitt (34a) aus einer Metalllegierung und der zweite Abschnitt (34b) aus Polymer, nämlich aus einem Thermoplast, hergestellt ist.

3. Medizinische Vorrichtung nach Anspruch 1 oder 2,
**dadurch gekennzeichnet,**
**dass** die Applikatorleitung (33) einen ersten Abschnitt (34a) mit einem ersten Durchmesser und einen zweiten Abschnitt (34b) mit einem zweiten Durchmesser umfasst, wobei sich der erste Durchmesser von dem zweiten Durchmesser unterscheidet, nämlich um mindestens 10 oder mindestens 20 oder mindestens 30 Prozent.

4. Medizinische Vorrichtung nach einem der vorhergehenden Ansprüche **dadurch gekennzeichnet, dass**
der Wasserstrahlapplikator (30) als Einweg-Applikator mit einer Düse mit einem Durchmesser von weniger als 0,5 Millimetern oder 0,2 Millimetern oder 0,12 Millimetern ausgebildet ist.

5. Medizinische Vorrichtung nach einem der vorherigen Ansprüche,
**dadurch gekennzeichnet, dass**
mindestens eine Befestigungsvorrichtung (50) dazu ausgebildet ist, den Applikatorauslass (31) in einer definierten Position, nämlich in Lateral- und Axial-Richtung, relativ zur zweiten Branche (12b) zu halten.

6. Medizinische Vorrichtung nach einem der vorherigen Ansprüche,
**dadurch gekennzeichnet, dass**
die Führung (40) einen Kanal (41) umfasst, der mindestens eine Seitenöffnung für die Sterilisation umfasst.

7. Medizinische Vorrichtung nach Anspruch 6,
**dadurch gekennzeichnet, dass**
eine Länge des Kanals maximal 80% der Branchenlänge der zweiten Branche (12b) ist.

8. Medizinische Vorrichtung nach einem der vorherigen Ansprüche,
**dadurch gekennzeichnet, dass**
das Instrument (10) eine Führungshülse (57), vorzugsweise aus Kunststoff, an einem der Handgriffe (14a, 14b) umfasst, wobei die Führungshülse (57) mindestens einen Abschnitt der Applikatorleitung (33) aufnimmt, um den Wasserstrahlapplikator (30) am Instrument (10) zu befestigen.

9. Medizinische Vorrichtung nach Anspruch 8,
**dadurch gekennzeichnet, dass**
die mindestens eine Befestigungsvorrichtung (50) einen ersten (45) und einen zweiten Anschlag (65) umfasst, um die Applikatorleitung (33) vorgespannt am Instrument (10) zu halten.

10. Medizinische Vorrichtung nach einem der vorherigen Ansprüche,
**dadurch gekennzeichnet, dass**
das Gelenk (11) ein Drehgelenk mit eine Rotationsachse umfasst, wobei die Medialbefestigungsvorrichtung (50) zur lösbaren Befestigung des Wasserstrahlapplikators (30) nahe bei oder auf der Rotationsachse angeordnet ist.

11. Medizinische Vorrichtung nach einem der vorherigen Ansprüche,
**dadurch gekennzeichnet, dass**
das Instrument (10) eine Schere ist, wobei die erste Branche (12a) ein erstes, gekrümmtes Scherenblatt und die zweite Branche (12b) ein zweites, gekrümmtes Scherenblatt umfasst.

12. Medizinische Vorrichtung nach einem der vorherigen Ansprüche,
**dadurch gekennzeichnet, dass**
das Instrument (10) eine elektrochirurgische Schere mit mindestens einem HF-Anschluss ist, wobei mindestens ein Abschnitt der Applikatorleitung (33), korrespondierend zu einer Isolierung des Scherenblatts eine Isolierung umfasst.

13. Medizinische Vorrichtung nach einem der vorherigen Ansprüche,
**dadurch gekennzeichnet, dass**
die Befestigungsvorrichtungen (50) eine Proximalbefestigungsvorrichtung (60) zur Herstellung einer Klippverbindung zu einem der Handgriffe (14a, 14b) in einem proximalen Bereich, nahe einem Scherenauge (16a, 16b) oder auf Höhe eines Scherenauges (16a, 16b) umfasst.

14. Verfahren zur Aufrüstung einer chirurgischen Schere zur Herstellung einer medizinischen Vorrichtung nach einem der Ansprüche 1 bis 13, umfassend die Schritte:
- Verbinden eines Applikatorauslasses (31) eines Wasserstrahlapplikators (30) mit einer Befestigungseinrichtung der Schere, wobei die Befestigungseinrichtung eine distale Öffnung umfasst;
- Anklippen oder Einrasten oder Schraubverbinden einer Applikatorleitung (33) an die Schere,
wobei die Applikatorleitung (33) umfasst:
- einen ersten Abschnitt (34a) mit einem ersten Elastizitätsmodul und einem ersten Durchmesser;
- einen zweiten Abschnitt (34b) mit einem zweiten Elastizitätsmodul und einem zweiten Durchmesser,
wobei sich der erste Elastizitätsmodul vom zweiten Elastizitätsmodul unterscheidet, wobei mindestens eine der Befestigungsvorrichtungen (50), nämlich eine Medialbefestigungsvorrichtung, eine fluide Verbindung zwischen dem ersten Abschnitt (34a) und dem zweiten Abschnitt (34b) herstellt.

## Claims

1. A medical device, comprising:
- an instrument (10);
- a water jet applicator (30) for transporting and delivering a fluid, comprising:
- an applicator line (33);
- a fluid terminal (36) for connecting the applicator line (33) to a fluid source;
- an applicator outlet (31) for delivering the fluid;
- at least two fastening devices for releasably fastening the water jet applicator (30) to an instrument (10),
wherein the applicator line (33) comprises:
- a first section (34a) having a first modulus of elasticity and a first diameter;
- a second section (34b) having a second modulus of elasticity and a second diameter,
wherein the first modulus of elasticity differs from the second modulus of elasticity,
wherein at least a first fastening device, i.e., a medial fastening device (50), establishes a fluidic connection between the first section (34a) and the second section (34b);
wherein the instrument (10) comprises a first branch (12a) and a second branch (12b), wherein the branches (12a, 12b) are interconnected via at least one articulation (11) and are operable by means of a first handle (14a) and a second handle (14b), respectively,
wherein at least a second fastening device comprises a distal guide (40) at one of the branches (12a, 12b) for receiving the applicator delivery (31) and a section of the applicator line (33).

2. The medical device according to claim 1,
**characterized in that**
the first section (34a) is made of a metal alloy, and the second section (34b) is made of polymer, i.e., of thermoplastic.

3. The medical device according to claim 1 or 2,
**characterized in that**
the applicator line (33) comprises a first section (34a) having a first diameter, and a second section (34b) having a second diameter, wherein the first diameter differs from the second diameter, i.e., by at least 10 or at least 20 or at least 30 percent.

4. The medical device according to any one of the preceding claims, **characterized in that**
the water jet applicator (30) is formed as a one-way applicator having a nozzle with a diameter of less than 0.5 millimeters or 0.2 millimeters or 0.12 millimeters.

5. The medical device according to any one of the preceding claims, **characterized in that**
at least one fastening device (50) is formed to hold the applicator outlet (31) in a defined position, i.e., in the lateral and axial direction, relative to the second branch (12b).

6. The medical device according to any one of the preceding claims, **characterized in that**
the guide (40) comprises a duct (41) comprising a lateral opening for sterilizing.

7. The medical device according to claim 6,
**characterized in that**
a length of the duct is at least 80% of the branch length of the second branch (12b).

8. The medical device according to any one of the preceding claims, **characterized in that**
the instrument (10) comprises a guiding sleeve (57), preferably of synthetic material, at one of the handles (14a, 14b), wherein the guiding sleeve (57) receives at least a section of the applicator line (33) in order to fasten the water jet applicator (30) to the instrument (10).

9. The medical device according to claim 8,
**characterized in that**
the at least one fastening device (50) comprises a first stop (45) and a second stop (65) for holding the applicator line (33) at the instrument (10) in a biased manner.

10. The medical device according to any one of the preceding claims, **characterized in that**
the articulation (11) is a rotational articulation having an axis of rotation, wherein the medial fastening device (50) for releasably fastening the water jet applicator (30) is arranged in the proximity of or on the axis of rotation.

11. The medical device according to any one of the preceding claims, **characterized in that**
the instrument (10) is scissors, wherein the first branch (12a) comprises a first, curved scissor blade, and the second branch (12b) comprises a second, curved scissor blade.

12. The medical device according to any one of the preceding claims, **characterized in that**
the instrument (10) is electrosurgical scissor having at least one HF terminal, wherein the section of the applicator line (33) comprises an isolation corresponding to an isolation of the scissor blade.

13. The medical device according to any one of the preceding claims, **characterized in that**
the fastening devices (50) comprise a proximal fastening device (60) for establishing a clip connection to one of the handles (14a, 14b) in a proximal range in the proximity of a scissors eye (16a, 16b) or on the level of a scissors eye (16a, 16b).

14. A method for upgrading a surgical scissors for producing a medical device according to any one of claims 1 to 13, comprising the steps of:
- connecting an applicator outlet (31) of a water jet applicator (30) to a fastening device of the scissors, wherein the fastening device comprises a distal opening;
- clipping or snapping or screw connecting an applicator line (33) to the scissors, wherein the applicator line (33) comprises:
- a first section (34a) having a first modulus of elasticity and a first diameter;
- a second section (34b) having a second modulus of elasticity and a second diameter,
wherein the first modulus of elasticity differs from the second modulus of elasticity, wherein at least one of the fastening devices (50), i.e., a medial fastening device, establishes a fluidic connection between the first section (34a) and the second section (34b).

## Revendications

1. Dispositif médical, comprenant :
- un instrument (10) ;
- un applicateur de jet d'eau (30) pour le transport et la délivrance d'un fluide, comprenant :
- une conduite d'applicateur (33) ;
- un raccord de fluide (36) destiné à relier la conduite d'applicateur (33) à une source de fluide ;
- une sortie d'applicateur (31) destinée à délivrer le fluide ;
- au moins deux dispositifs de fixation destinés à fixer de manière détachable l'applicateur de jet d'eau (30) à un instrument (10),
sachant que la conduite d'applicateur (33) comprend :
- une première section (34a) présentant un premier module d'élasticité et un premier diamètre,
- une deuxième section (34b) présentant un deuxième module d'élasticité et un deuxième diamètre,
sachant que le premier module d'élasticité se distingue du deuxième module d'élasticité,
sachant qu'au moins un premier dispositif de fixation, à savoir un dispositif de fixation médical (50), établit une liaison fluidique entre la première section (34a) et la deuxième section (34b),
sachant que l'instrument (10) comprend une première branche (12a) et une deuxième branche (12b), sachant que les branches (12a, 12b) sont reliées entre elles par au moins une articulation (11) et sont actionnables moyennant une première poignée (14a) et une deuxième poignée (14b) respectivement,
sachant qu'au moins un deuxième dispositif de fixation comprend un guidage distal (40) au niveau d'une des branches (12a, 12b), destiné à recevoir la sortie d'applicateur (31) et une section de la conduite d'applicateur (33).

2. Dispositif médical selon la revendication 1,
**caractérisé en ce que**
la première section (34a) est composée d'un alliage métallique et la deuxième section (34b), de polymère, à savoir d'un thermoplastique.

3. Dispositif médical selon la revendication 1 ou 2,
**caractérisé en ce que**
la conduite d'applicateur (33) comprend une première section (34a) présentant un premier diamètre et une deuxième section (34b) présentant un deuxième diamètre, sachant que le premier diamètre se distingue du deuxième diamètre, à savoir à raison d'au moins 10 ou d'au moins 20 ou d'au moins 30 pour cent.

4. Dispositif médical selon l'une des revendications précédentes,
**caractérisé en ce que**
l'applicateur de jet d'eau (30) est constitué comme applicateur à usage unique doté d'une tuyère présentant un diamètre de moins de 0,5 millimètre ou 0,2 millimètre ou 0,12 millimètre.

5. Dispositif médical selon l'une des revendications précédentes,
**caractérisé en ce que**
au moins un dispositif de fixation (50) est constitué pour maintenir la sortie d'applicateur (31) dans une position définie, à savoir en direction latérale et axiale, par rapport à la deuxième branche (12b).

6. Dispositif médical selon l'une des revendications précédentes,
**caractérisé en ce que**
le guidage (40) comprend un canal (41) qui comprend au moins une ouverture latérale pour la stérilisation.

7. Dispositif médical selon la revendication 6,
**caractérisé en ce que**
une longueur du canal est de maximum 80 % de la longueur de branche de la deuxième branche (12b).

8. Dispositif médical selon l'une des revendications précédentes,
**caractérisé en ce que**
l'instrument (10) comprend une douille de guidage (57), de préférence en matière synthétique, au niveau d'une des poignées (14a, 14b), sachant que la douille de guidage (57) reçoit au moins une section de la conduite d'applicateur (33) pour fixer l'applicateur de jet d'eau (30) à l'instrument (10).

9. Dispositif médical selon la revendication 8,
**caractérisé en ce que**
l'au moins un dispositif de fixation (50) comprend une première (45) et une deuxième butée (65) pour maintenir la conduite d'applicateur (33) précontrainte au niveau de l'instrument (10).

10. Dispositif médical selon l'une des revendications précédentes,
**caractérisé en ce que**
l'articulation (11) comprend une articulation rotative présentant un axe de rotation, sachant que le dispositif de fixation médical (50) destiné à fixer de manière détachable l'applicateur de jet d'eau (30) est disposé près de ou sur l'axe de rotation.

11. Dispositif médical selon l'une des revendications précédentes,
**caractérisé en ce que**
l'instrument (10) est des ciseaux, sachant que la première branche (12a) comprend une première lame de ciseaux courbée et la deuxième branche (12b), une deuxième lame de ciseaux courbée.

12. Dispositif médical selon l'une des revendications précédentes,
**caractérisé en ce que**
l'instrument (10) est des ciseaux électrochirurgicaux dotés d'au moins un raccord HF, sachant qu'au moins une section de la conduite d'applicateur (33) comprend une isolation, de manière correspondante à une isolation de la lame de ciseaux.

13. Dispositif médical selon l'une des revendications précédentes,
**caractérisé en ce que**
les dispositifs de fixation (50) comprennent un dispositif de fixation proximal (60) destiné à établir une liaison par clipsage avec une des poignées (14a, 14b) dans une zone proximale, près d'un anneau de ciseaux (16a, 16b) ou à hauteur d'un anneau de ciseaux (16a, 16b).

14. Procédé destiné à équiper des ciseaux chirurgicaux afin de réaliser un dispositif médical selon l'une des revendications 1 à 13, comprenant les étapes :
- liaison d'une sortie d'applicateur (31) d'un applicateur de jet d'eau (30) avec un dispositif de fixation des ciseaux, sachant que le dispositif de fixation comprend une ouverture distale ;
- clipsage ou encliquetage ou liaison par vissage d'une conduite d'applicateur (33) aux ciseaux,
sachant que la conduite d'applicateur (33) comprend :
- une première section (34a) présentant un premier module d'élasticité et un premier diamètre ;
- une deuxième section (34b) présentant un deuxième module d'élasticité et un deuxième diamètre,
sachant que le premier module d'élasticité se distingue du deuxième module d'élasticité, sachant qu'au moins un des dispositifs de fixation (50), à savoir un dispositif de fixation médical, établit une liaison fluidique entre la première section (34a) et la deuxième section (34b).
